**Europäisches Patentamt**

**European Patent Office**

**Office européen des brevets**

(19)

(11) Veröffentlichungsnummer: **0 386 241**
**A1**

(12) **EUROPÄISCHE PATENTANMELDUNG**
veröffentlicht nach Art. 158 Abs. 3
EPÜ

(21) Anmeldenummer: **88909403.3**

(22) Anmeldetag: **23.08.88**

(86) Internationale Anmeldenummer:
**PCT/SU88/00164**

(87) Internationale Veröffentlichungsnummer:
**WO 90/02353 (08.03.90 90/06)**

(51) Int. Cl.5: **G02B 23/00, A61N 5/01**

(43) Veröffentlichungstag der Anmeldung:
**12.09.90 Patentblatt 90/37**

(84) Benannte Vertragsstaaten:
**CH DE FR GB IT LI**

(71) Anmelder: **RADIOTEKHNICHESKY INSTITUT IMENI AKADEMIKA A.L.MINTSA AKADEMII NAUK SSSR**
**ul. 8 Marta, 10-12**
**Moscow, 125083(SU)**

(72) Erfinder: **LOZHENKO, Alexandr Sergeevich**
**Nadsonovsky tupik, 5-32 Moskovskaya obl.**
**Pushkino, 141200(SU)**
Erfinder: **RECHITSKY, Vladimir Iliich**
**Peschany per., 8-18**
**Moscow, 125252(SU)**
Erfinder: **SHENDALEV, Viktor Nikolaevich**
**ul. Vucheticha, 4-30**
**Moscow, 125206(SU)**

(74) Vertreter: **Lehn, Werner, Dipl.-Ing. et al**
**Hoffmann, Eitle & Partner Patentanwälte**
**Arabellastrasse 4**
**D-8000 München 81(DE)**

(54) **LICHTFÜHRENDE ANORDNUNG FÜR MEDIZINISCHE BEHANDLUNGEN.**

(57) Die Lichtleitvorrichtung für Lichttherapie enthält einen Faser-Lichtleiter (1) und ein lichtstreuendes Element (5) an dessen Distalende. Die Schutzhülle (4) des Lichtleiters (1) ist über die Stirnseite (6) dessen Distalendes hinausragend ausgebildet und bildet mit dieser Stirnseite (6) einen geschlossenen Raum, der vollständig mit optisch trübem Medium (7) ausgefüllt ist, das als lichtstreuendes Element (5) auftritt.

FIG. 1

## LICHTLEITVORRICHTUNG FÜR LICHTTHERAPIE

### Technisches Gebiet

Die vorliegende Erfindung bezieht sich auf Lichtleitvorrichtungen zur Übertragung von Strahlungsenergie, insbesondere auf Lichtleitvorrichtungen für Lichttherapie.

### Zugrundeliegender Stand der Technik

Bekannt ist eine Lichtleitvorrichtung für Lichttherapie, die einen Faser-Lichtleiter und ein lichtstreuendes Element enthält, das als entisolierte Lichtführende Ader des Lichtleiters ausgebildet ist, deren Oberfläche mit Flußsäure behandelt würde (Optics and laser technology, 1984, February, H.Fujll and other, "Light seattering properties of a rough-ended optical fibre", p. 40 - 41).

Die erwähnte Vorrichtung ist nicht anwendbar, wenn Höhlen laser-therapeutisch zu behandeln sind, weil eine große Gefahr besteht, daß das lichtsteuende Element als spröde Baugruppe im Patientenkörper bricht.

Es ist eine Lichtleitvorrichtung für Lichttherapie bekannt, die einen Faser-Lichtleiter mit einer lichtführenden Ader und einer Lichtreflexions- und einer Schutzhülle sowie ein lichtstreuendes Element enthält, das am Distalende des Faser-Lichtleiters montiert ist (Lasers in Surgery and Medicine, No. 6, 1986, M Arnfield and other "Cylindrical Irradiator Fiber Tip för Photodynamic Therapy, P. 151).

Bei der besagten Lichtleitvorrichtung für Lichttherapie ist das lichtstreuende Element als Becher ausgebildet, bei dem die Stirnseiten seiner Wände am entisolierten Bereich der lichtführenden Ader des Faser-Lichtleiters angeklebt sind, während die Innenfläche der Becherwände mit Epoxidharz beschichtet ist.

Die erwähnte Vorrichtung kann bei den laser-therapeutischen Behandlungen der Körperhöhlen keine Sicherheit für den Patienten gewährleisten, da sie traumatisch wirken kann.

- 2 -

Diese Vorrichtung ist kompliziert bei der Herstellung . Überdies ermöglicht die Vorrichtung nur eine einzige Art der Lichtverteilungsindikatrix der Strahlung, d.h., es wird keine Anpassung der Form der Lichtverteilungsindikatrix der Strahlung an die Form des pathologischen Herdes erreicht und somit der Behandlungsfaktor nicht optimiert.

Offenbarung der Erfindung

Der vorliegenden Erfindung liegt die Aufgabe zugrunde, eine Lichtleitvorrichtung für Lichttherapie zu schaffen, bei welcher die Schutzhülle des Faser-Lichtleiters in Verbindung mit dem Lichtstreuernden Element so ausgeführt wären, daß für den Patienten absolut keine traumatische Gefahr bei der Lichttherapie besteht und der Behandlungsfaktor durch die Anpassung der Form der Lichtverteilungsindikatrix der Strahlung an die Form des pathologischen Herdes optimiert werden könnte, wobei die konstruktive Ausführung der Lichtleitvorrichtung einfach wäre.

Das wird dadurch erreicht, daß bei der Lichtleitvorrichtung für Lichttherapie, die einen Faser-Lichtleiter mit einer lichtführenden Ader und einer Lichtreflexions- und einer Schutzhülle sowie ein am Distalende des Faser-Lichtleiters montiertes lichtstreuendes Element enthält, die Schutzhülle des Faser-Lichtleiters erfindungsgemäß über die Stirnseite des Distalendes der Faser-Lichtleiters hinausragend ausgebildet ist und mit dieser Stirnseite einen geschlossenen Raum bildet, während das lichtstreuernde Element aus optisch trübem Medium besteht, das mit der Stirnseite des Faser-Lichtleiters in Berührung steht und den gesamten gebildeten geschlossenen Raum ausfüllt.

Diese konstruktive Ausführung der Lichtleitvorrichtung für Lichttherapie erlaubt bei laser-therapeutischen Behandlungen der Körperhöhlen die Gefahr traumatischer

- 3 -

Verletzungen vollkommen auszuschließen, d.h., es wird
eine hohe Betriebszuverlässigkeit erreicht und der Behandlungsfaktor bei einfacher baulicher Gestaltung der
Vorrichtung optimiert.

Kurze Beschreibung der Zeichnungen

Nachfolgen wird die Erfindung durch Beschreibung eines Ausführungsbeispiel derselben und beigelegte Zeichnungen näher erläutert, in welchen es zeigen:

Fig. 1 eine erfindungsgemäße Lichtleitvorrichtung
für Lichttherapie (im Längsschnitt);

Fig. 2 Lichtverteilungsindikatrizen in Form von Ellipsoiden und eiener Kugel, die mit der Lichtleitvorrichtung nach Fig. 1 erhalten wurden;

Fig. 3 die Lichtverteilungsindikatrix in Form eines
Torus, die mit der Lichtleitvorrichtung nach Fig. 1 erhalten wurde.

Beste Ausführungsform der Erfindung

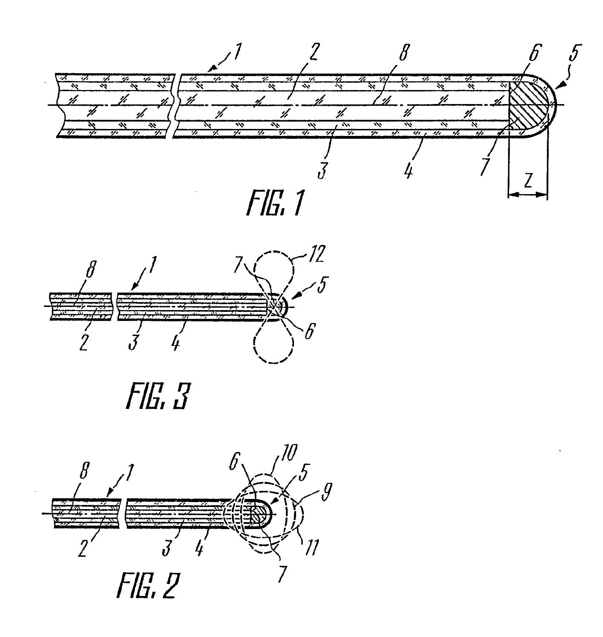

Die erfindungsgemäße Lichtleitvorrichtung für Lichttherapie enthält einen Faser-Lichtleiter 1 (Fig. 1) mit
einer lichtführenden Ader 2 und mit einer Lichtreflexions-
3 und einer Schutzhülle 4 sowie ein lichtstreuendes Element 5, das am Distalende des Faser-Lichtleiters 1 montiert
ist.

Die Schutzhülle 4 ist über die Stirnseite 6 des Distalendes des Faser-Lichtleiters 1 hinausragend ausgebildet und bildet zusammen mit dieser Stirnseite 6 einen
geschlossenen Raum. Das lichtstreuende Element 5 besteht
aus einem optisch trüben Medium 7, das mit der Stirnseite
6 des Faser-Lichtleiters 1 in Berührung steht und den
gesamten gebildeten geschlossenen Raumausfüllt.

Als optisch trübes Medium kann ein Klebstoff auf Basis von Epoxid-, niedermolekularen oder Polyamidharzen
verwendet werden.

- 4 -

Die Funktiosweise der erfindungsgemäßen Lichtleit -
vorrichtung für Lichttherapie besteht im folgenden.

Die Strahlung eines (in der Zeichnung nicht dargestellten) Lasers breitet sich über die lichtführende
Ader 2 (Fig. 2) des Faser-Lichtleiters 1 aus und gelangt
in das lichtstreuernde Element 5, wo sie im optisch trüben Medium 7 zerstreut wird. Die Streuestrahlung tritt
über die Schutzhülle 4 am Distalende des Lichtleiters
1 der erfindungsgemäßen Vorrichtung aus.

Je nach dem Dämpfungsfaktor der Strahlung im optisch
trüben Medium 7 und der Länge des lichtstreuenden 5
Elementes entlang der optischen Achse 8 des Lichtleiters
1 können verschiende Formen der Lichtverteilungsindikatrizen der Strahlung enthalten werden.

So kann die Länge z des lichtstreuernden Elementes
5 entlang der optischen Achse 8 des Lichtleiters 1 je
nach dem Dämpfungsfaktor $\varepsilon$ des optisch trüben Mediums
7 des erwähnten Elementes 5 gewährleisten, daß verschiedene Lichtverteilungsindikatrizen der Strahlung bei deren
Gleichmäßigkeit erhalten werden, die für die Durchführung
der laser-therapeutischen Bahandlungen ausreichend effektiv ist.

Bei $z < 1/\varepsilon$   bzw. $z > 1/\varepsilon$   haben die Licht-
verteilungsindikatrizen 9, 10 und 11 (Fig. 2) der Strahlung die Form von einer Kugel bzw. Ellipsoiden. Diese
Art der Indikatrizen wird für die Licht therapie bei
Lungenabszess, Pleuraempyem, Highmoritis, chronischen
gastroduodenalen Geschwüren breit verwendet.

Bei $z = 1/\varepsilon$   hat die Lichtverteilungsindikatrix 12
(Fig. 3) der Strahlung die Form eines Torus. Diese Art
der Indikatrix wird bei Lichttherapie von rohrförmigen
Höhlen mit lokalisierten pathologischen  Herden an den
Wänden verwendet, z.B. bei Verätzungen der Speiseröhre,
Harnröhrenentzündungen, unspezifischer Urethritis.

Durch die Möglichkeit, die vorgegebene Art der Lichtverteilungsindikatrizen  der Strahlung zu erhalten, wer-

- 5 -

den die optimale Belichtung und die maximale Anwendung der Strahlung als Bahandlungsfaktor erreicht.

Die erfindungsgemäße Vorrichtung ist somit zur ummittelbaren Einwirkung mit Laser-Strahlung auf pathologische Herde anwendbar, die in schwerzugänglichen rohrförmigen Höhlen des Patientenkörpers lokalisiert sind, die geringe Abmessungen von 1,2 bis 1,8 mm aufweisen, z.B. Wundflächen und postoperative Nähte auf dem Harn- leiter, ohrtrompetenentzündung, Pleuraempyem, Lungen- abszeß.

Die erfindungsgemäße Lichtleitvorrichtung für Lichttherapie zeichnet sich außerdem durch eine hohe Betriebszuverlässigkeit und eine einfache konstruktive Ausführung aus.

Gewerbliche Verwertbarkeit

Die Lichtleitvorrichtung für Lichttherapie kann in der Medizin erfolgreich angewendet werden, wenn laser- -therapeutische Prozeduren in inneren Höhlen unter Lokalisierung von pathologischen Herden in schwerzugänglichen Höhlen des Patientenkörpers durchzuführen sind.

- 6 -

PATENTANSPRUCH

Lichtleitevorrichtung für Lichttherapie, die einen Faser-Lichtleiter (1) mit einer lichtführenden Ader (2) und einer Lichtreflexions- (3) und einer Schutzhülle (4) sowie ein am Distalende des Faser-Lichtleiters (1) montiertes lichtstreuendes Element (5) enthält, dadurch g e k e n n z e i c h n e t, daß die Schutzhülle (4) des Faser-Lichtleiters (1) über die Stirnseite (6) des Distalendes des Faser-Lichtleiters (1) hinausragend ausgebildet ist und mit dieser Stirnseite (6) einen geschlossenen Raum bildet, während das lichtstreuende Element (5) aus Optisch trübem Medium (7) besteht, das mit der Stirnseite (6) des Faser-Lichtleiters (1) in Berührung steht und den gesamten gebildeten geschlossenen Raum ausfüllt.

FIG. 1

FIG. 3

FIG. 2

## I. CLASSIFICATION OF SUBJECT MATTER (if several classification symbols apply, indicate all)

According to International Patent Classification (IPC) or to both National Classification and IPC

IPC⁴    G02B 23/00, A61N 5/01

## II. FIELDS SEARCHED

### Minimum Documentation Searched ⁷

| Classification System | Classification Symbols |
|---|---|
| IPC⁴ | G02B 23/00, A61N 5/01 |

### Documentation Searched other than Minimum Documentation
to the Extent that such Documents are included in the Fields Searched ⁸

## III. DOCUMENTS CONSIDERED TO BE RELEVANT ⁹

| Category * | Citation of Document, ¹¹ with indication, where appropriate, of the relevant passages ¹² | Relevant to Claim No. ¹³ |
|---|---|---|
| A | GB, B, 1393699 (SPOT-SHOT LIMITED) 14 May 1975 (14.05.75) see figure 1 | 1 |
| A | CH, A5, 626454 (DANIEL LOUIS LEGRAND) 13 November 1981 (13.11.81) see figure 1 | 1 |
| A | DE, B2, 2544519 (OLYMPUS OPTICAL CO.LTD ) 10 July 1980 (10.07.80) see figures 1,3;column 3,lines 5-20 | 1 |
| A | DE, A1, 2911478 (EICHLER JüRGEN) 25 September 1980 (25.09.80)  see figure 2 | 1 |
| A | DE, A1, 3113869 (ASAHI KOGAKU KOGYO K.K.) 28 January 1982 (28.01.82) see figure 2 | 1 |

* Special categories of cited documents: ¹⁰

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 14 April 1989 (14.04.89) | 23 May 1989 (23.05.89) |

| International Searching Authority | Signature of Authorized Officer |
|---|---|
| ISA/SU | |